# EUROPEAN PATENT APPLICATION

(11) **EP 4 473 973 A1**
(43) Date of publication of application: **11.12.2024**
(21) Application number: 22928971.5
(22) Date of filing: 26.12.2022
(51) Int. Cl.: A61K 38/10, A61P 35/04, C07K 7/08

(54) **VIPR2 ANTAGONIST PEPTIDE FOR SUPPRESSING CANCER METASTASIS**

(30) Priority: 25.02.2022 JP 2022027791
(71) Applicant: Ichimaru Pharcos Co., Ltd., Motosu-shi, Gifu 501-0475 (JP); Hiroshima University, Higashi-Hiroshima-shi Hiroshima 739-8511 (JP)
(72) Inventor: AGO Yukio, Hiroshima-shi, Hiroshima 739-8511 (JP); ASANO Satoshi, Hiroshima-shi, Hiroshima 739-8511 (JP); SAKAMOTO Kotaro, Motosu-shi, Gifu 501-0475 (JP)
(74) Representative: Hasegawa, Kan
(86) International application number: PCT/JP2022/047969
(87) International publication number: WO 2023/162456

(57) **Abstract**

Disclosed is a means for suppressing cancer metastasis is disclosed by examining the role of a VIPR2 signal affecting migration of cancer cells and its mechanism. Provided is a composition for suppressing cancer metastasis, containing: a cyclic peptide having an amino acid sequence represented by the formula (1): c[X¹-Pro²-X³-Tyr⁴-Leu⁵-Pro⁶-c(X⁷-X⁸-Leu⁹-Cys¹⁰]-Xⁿ)-X¹²-X¹³ (1) (X¹, X³, X⁷, X⁸, X¹¹, X¹², and X¹³ are as described in the specification.); a derivative or a modified form of the cyclic peptide; or a pharmacologically acceptable salt thereof.

## Description

### CROSS-REFERENCE TO RELATED APPLICATIONS

This application claims priority to Japanese Patent Application No. 2022-027791 filed in Japan on February 25, 2022, the entire disclosure of which is incorporated herein by reference.

### TECHNICAL FIELD

The present invention relates to a composition for suppressing cancer metastasis, and more specifically to a composition comprising a VIPR2 antagonist peptide for suppressing cancer metastasis.

### BACKGROUND ART

Cell migration is an evolutionarily conserved mechanism and plays a role not only in normal processes such as embryonic development, immunity, angiogenesis, and wound healing, but also in pathogenic processes such as cancer metastasis. The cell migration is controlled by the localization of actin filaments in the cell, and a protruding structure (protrusion) called lamellipodia is formed at the front edge of the cell. Extracellular stimulation by growth factors, cytokines, chemokines, etc., induces the formation of lamellipodia. For example, in the case of signaling via chemokines and G protein-coupled receptor (GPCR) as a receptor of chemokine, GTP-bound Gαi directly activates SRK-like kinase and upregulates a phosphatidylinositol 3-kinase (PI3K) signal. Activated PI3K converts phosphatidylinositol 4,5-diphosphate[PI(4,5)P₂] to phosphatidylinositol 3,4,5-triphosphate[PI(3,4,5)P₃]. PI(3,4,5)P₃ promotes phosphorylation of AKT, promotes movement of verprolin homologous protein 2 (WAVE2) of the WASP family to the cell membrane, and controls remodeling of actin filaments via GEF-Rac, thereby promoting the formation of lamellipodia. Indeed, gain-of-function mutations in PI3K (i.e. mutation of the p110 catalytic subunit of PI3K) enhance the PI(3,4,5)P₃ signaling pathway and are frequently found in breast cancer and the like. The activated PI(3,4,5)P₃ pathway induces growth and motility of cancer cells, resulting in enhanced movement and invasion of the cancer cells.

Vasoactive intestinal peptide (VIP), which is a neuropeptide closely related to pituitary adenylate cyclase-activating polypeptide (PACAP), is widely expressed in the central and peripheral nervous systems. G protein-coupled receptors (GPCR), VIPR1 and VIPR2 (also referred to as VPAC1 and VPAC2), which are receptors thereof, are widely expressed in the brain, but are also expressed in many peripheral target organs such as the cardiovascular system, the renal system, the digestive system, the immune system, the endocrine system, and the reproductive system. There are many reports on the relationship between VIP and tumor proliferation. Of note, increased expression of VIPR2 mRNA and/or the increased copy number of the VIPR2 genes has been reported in several types of cancers, such as ovarian epithelial tumor, glioblastoma, and invasive breast cancer. However, the pathophysiological role and the like of VIPR2 in cancer are still largely unknown.

The present inventors have found a novel group of VIPR2 antagonist peptides and disclosed them (see NON-PATENT DOCUMENT 1, and PATENT DOCUMENT 1). Among these, a representative bicyclic peptide, KS-133 (disclosed in NON-PATENT DOCUMENT 2; referred to as Seq-10 (SEQ ID NO: 2) in the Examples described below), has also been reported to suppress cognitive decline in psychiatric disease model mice (see NON-PATENT DOCUMENT 2).

### CITATION LIST

### NON-PATENT DOCUMENTS

NON-PATENT DOCUMENT 1: Sakamoto K et al., Biochemistry Biophys Res Commun (2018), 503:1973-1979.
NON-PATENT DOCUMENT 2: Sakamoto K et al., Frontiers in Pharmacology (2021), 12:751587
NON-PATENT DOCUMENT 3: Biochemical and Biophysical Research Communications 552 (2021) 106-113

### PATENT DOCUMENT

PATENT DOCUMENT 1: International Patent Publication No. WO 2021/200259

### SUMMARY OF THE INVENTION

### TECHNICAL PROBLEM

The problem to be solved by the present invention is to provide a means for suppressing cancer metastasis, for example, by examining the role of the VIPR2 signal affecting migration of cancer cells and its mechanism.

### SOLUTION TO THE PROBLEM

The present invention has been made to solve the above-described problem, based on the discovery that a VIP-VIPR2 signal controls localization and extension of actin for lamellipodia formation by WAVE2 via synthesis of PI(3,4,5)P₃, thereby controlling migration of cancer cells in vitro and in vivo. That is, the present invention provides a means for suppressing cancer metastasis by inhibiting the function of VIPR2, and specifically encompasses the following embodiments.

A composition for suppressing cancer metastasis, the composition containing a cyclic peptide having an amino acid sequence represented by the formula (1):

c[X¹-Pro²-X³-Tyr⁴-Leu⁵-Pro⁶⁻c(X⁷-X⁸-Leu⁹-Cys¹⁰]-X¹¹)-X^{I2}-X¹³ (1)

where X¹ represents cysteine, Mpa (3-mercaptopropionic acid), or D-cysteine, X³ represents N-methylated glycine, N-methylated alanine, 2-azetidine-2-carboxylic acid, proline, hydroxyproline, 3,4-dehydroproline, pipecolic acid, serine, or lysine, X⁸ represents tyrosine, proline, or arginine, X⁷ and X¹¹ represent any combination of lysine and aspartic acid, ornithine and glutamic acid, aspartic acid and lysine, glutamic acid and ornithine, lysine and glutamic acid, or glutamic acid and lysine, X¹² and X¹³ each independently represent leucine, isoleucine, or norleucine, X¹ and Cys¹⁰ form a disulfide bond between their side chains, and X⁷ and X¹¹ form an amide bond between their side chains, whereby the peptide of the formula (1) has two cyclic structures in a molecule, and an N-terminus amino group and a C-terminus carboxy group is optionally modified or deleted; a derivative or a modified form of the cyclic peptide; or a pharmacologically acceptable salt thereof.

In one preferred embodiment, in the cyclic peptide of the formula (I), X¹ represents cysteine, X³ represents proline or serine, X⁷ represents lysine, X⁸ represents tyrosine, X¹¹ represents aspartic acid, X¹² and X¹³ each independently represent leucine, isoleucine, or norleucine, the N-terminus amino group is acetylated, and the C-terminus carboxy group is amidated.

The composition can be used, for example, for treating a subject containing cancer cells in which signaling pathways downstream of PI3K such as AKT or WAVE2 are activated via VIPR2, and can also be used for suppressing phosphorylation of AKT in cancer cells via VIPR2.

### ADVANTAGES OF THE INVENTION

The composition containing the cyclic peptide according to the present invention can suppress cancer metastasis, for example, by inhibiting the function of VIPR2.

### BRIEF DESCRIPTION OF THE DRAWINGS

FIGS. 1A to 1C show results of examination of the influence of VIP on the phosphorylation of AKT in MCF-7 and MDA-MB-231 breast cancer cells.
FIGS. 2A to 2C show results of evaluation of cell motility in MCF-7 breast cancer cells overexpressing MCF-7 and VIPR2-EGFP.
FIG. 3 shows results of measurement of migration movement of MDA-MB-231 breast cancer cells overexpressing EGFP or VIPR2-EGFP using a transwell migration assay.
FIG. 4 shows result of quantification of the area of lamellipodia.
FIGS. 5A and 5B show results of in vivo evaluation of the migration activity of MDA-MB-231 breast cancer cells overexpressing VIPR2-EGFP.

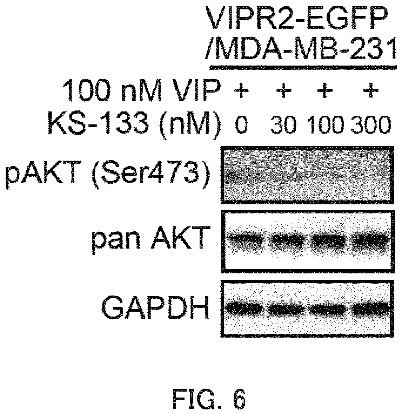

FIG. 6 shows results of evaluation of AKT phosphorylation after adding a VIPR2-selective antagonist peptide to MDA-MB-231 breast cancer cells overexpressing VIPR2-EGFP.
FIG. 7 is a schematic diagram of the structure of a transwell used for evaluation of the cell motility in MDA-MB-231 breast cancer cells overexpressing VIPR2-EGFP.
FIGS. 8A and 8B show results of evaluation of the effect of a VIPR2-selective antagonist peptide for suppressing the migration of MDA-MB-231 breast cancer cells overexpressing VIPR2-EGFP.

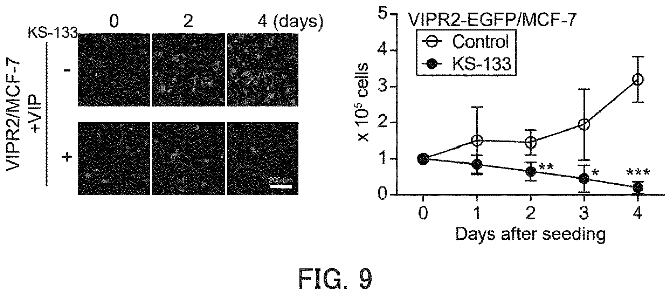

FIG. 9 shows results of confirmation of cell proliferation effect when KS-133 as a VIPR2 antagonist was added (KS-133 group) or when KS-133 was not added (control group) in MCF-7 cells overexpressing VIPR2. The confirmation was performed in each group (Control group, KS-133), N=4. The numerical values of the graph (mean values of each group) are shown in Table 3. Data are shown as mean ± SD. *: p<0.05, **: p<0.01, ***: p<0.001 compared to Day 0 (Mann-Whitney U test). In the MCF-7 cells overexpressing VIPR2, cell proliferation was significantly suppressed from Day 2 of culture or later by the addition of KS-133. In the control group, cell proliferation was confirmed over time.

### DESCRIPTION OF EMBODIMENTS

Embodiments of the present invention will be described below with reference to the drawings. The embodiments described below do not limit the claimed invention. All the components described in the embodiments and combinations thereof are not necessarily essential to achieve the present invention.

### (Definitions)

As used herein, a peptide refers to two or more amino acids bonded together by amide bonds (peptide bonds), and can be, for example, 2 to 20 amino acids bonded together by amide bonds. The left end is the N-terminus (amino terminus) and the right end is the C-terminus (carboxy terminus) according to the peptide notation. The first carbon atom adjacent to the carbonyl group forming the peptide bond is referred to as the Cα carbon.

As used herein, "any amino acid or derivative thereof' is used in its broadest sense and encompasses, in addition to natural amino acids, artificial amino acids having non-natural structures, chemically synthesized compounds having properties known in the art that are characteristics of amino acids, and also carboxylic acids having functional groups. Examples of unnatural amino acids include, but are not limited to, a D-amino acid, an α/α-disubstituted amino acid with a main chain structure different from those of natural amino acids (e.g., α-methylated amino acids such as 2-aminoisobutyric acid), an N-alkyl-amino acid (e.g., N-methylated amino acid), an N-substituted glycine (peptoid), an amino acid with an extended main chain (e.g., β homoamino acid and γ homoamino acid), an amino acid with a side chain structure different from those of natural amino acids (e.g., cyclohexylalanine, allylglycine, 2-(2-pyridyl)-glycine, and 3-(1H-benzimidazol-2-yl)-alanine), an amino acid with a partially substituted side chain (e.g., norleucine, diaminopropanoic acid, and 3-(2-pyridyl)-alanine), an amino acid with an extra functional group in a side chain; an amino acid with an extra C, alkyl group, or a methyl group in a side chain (e.g., homonorleucine and γ-methylleucine), an amino acid with a halogen atom (F, Cl, Br, I) in a side chain (e.g., 3-chloro-alanine), a carboxylic acid with a halogen atom (F, Cl, Br, I) in a side chain (e.g., 3-chloropropanoic acid), a carboxylic acid with a functional group in a side chain (e.g., 3-butenoic acid), an amino acid with an extra N or an amino group in a side chain (e.g., β-azidoalanine, ornithine), an amino acid with an extra O or a methoxy group in a side chain (e.g., O-methyl-serine, O-methyl-threonine), an amino acid with an extra hydroxy group in a side chain (e.g., 3-hydroxy-phenylalanine), an amino acid with an extra carboxy group (-COOH) in a side chain (e.g., 3-carboxyphenylalanine), an amino acid with an extra S in a side chain (e.g., ethionine), an amino acid in which a carboxylic acid functional group in a side chain is protected by esters (e.g., aspartic acid-4-methyl ester), and an amino acid in which a thio group (-S-) in a side chain has been oxidized and converted to a sulfinyl group (-S(=O)-) or a sulfonyl group (-S(=O)₂-) (methionine sulfoxide).

As used herein, "VIPR2" means "vasoactive intestinal peptide receptor 2", which is a protein of mammals such as mouse, rat, dog, monkey, and human.

As used herein, "suppressing cancer metastasis" means, for example, either or both of suppressing metastasis of a tumor in a case where a primary tumor has not metastasized yet, or suppressing additional metastasis of a tumor in a case where the tumor has already metastasized. Metastasis of cancer cells refers to the movement of cancer cells from a place where the cancer cells originate (primary lesion) to form a tumor again at a distant site. There have been various research reports on the mechanism, and the mechanism includes, for example, processes such as 1) proliferation of cancer cells in a primary lesion, 2) detachment of cancer cells from a primary lesion and infiltration into a vessel (blood vessel or lymphatic vessel), 3) movement within a vessel, 4) adhesion of a metastatic organ to vascular endothelium, 5) infiltration into a metastatic organ, and 6) proliferation within a metastatic organ. Cell migration plays an important role in the movement of cancer cells in these processes, and it is considered that cancer metastasis can be suppressed by inhibiting the cell migration.

### (Cyclic Peptide)

A cyclic peptide as one active ingredient of the present invention is disclosed in PATENT DOCUMENT 1, and the entire contents thereof are incorporated herein by reference. The peptide disclosed in PATENT DOCUMENT 1 is stabilized by bicyclization while maintaining or enhancing the characteristics (pharmacophore) related to the VIPR2 binding activity of VIpep-3 disclosed in NON-PATENT DOCUMENT 1. Any of the peptides disclosed in these documents can be used for the novel use of the present invention.

Among these cyclic peptides, a cyclic peptide of an embodiment particularly suitable for suppressing cancer metastasis having an amino acid sequence represented by Formula (1) below:

c[X¹-Pro²-X³-Tyr⁴-Leu⁵-Pro⁶-c(X⁷-X⁸-Leu⁹-Cys¹⁰]-X¹¹)-X¹²-X¹³ (1).

In Formula (1), X¹ represents cysteine, Mpa (3-mercaptopropionic acid) or D-cysteine, X³ represents N-methylated glycine, N-methylated alanine, 2-azetidine-2-carboxylic acid, proline, hydroxyproline, 3,4-dehydroproline, pipecolic acid, serine or lysine, X⁸ represents tyrosine, proline, or arginine, X⁷ and X¹¹ represent any combination of lysine and aspartic acid, ornithine and glutamic acid, aspartic acid and lysine, glutamic acid and ornithine, lysine and glutamic acid, or glutamic acid and lysine, X¹² and X¹³ each independently represent leucine, isoleucine or norleucine, X¹ and Cys¹⁰ form a disulfide bond between their side chains, and X⁷ and X¹¹ form an amide bond between their side chains, whereby the peptide of the formula (1) has two cyclic structures in the molecule, and an N-terminus amino group and a C-terminus carboxy group can be modified or deleted.

In a cyclic peptide of a further preferred embodiment, in the formula (1), X¹ represents cysteine, X³ represents proline or serine, X⁷ represents lysine, X⁸ represents tyrosine, X¹¹ represents aspartic acid, X¹² and X¹³ each independently represent leucine, isoleucine, or norleucine. The N-terminus amino group is acetylated and the C-terminus carboxy group is amidated.

Examples of the individual cyclic peptide included in the above formula (1) include the following: c(Mpa-Pro-Pro-Tyr-Leu-Pro-c[Lys-Tyr-Leu-Cys)-Asp]-Leu-Ile-NH₂ (SEQ ID NO: 1); Ac-c[Cys¹-Pro²-Pro³-Tyr⁴-Leu⁵-Pro⁶-c(Lys⁷-Tyr⁸-Leu⁹-Cys¹⁰]-Asp¹¹)-Leu¹²-Ile¹³-NH₂ (SEQ ID NO: 2); and the peptides described in paragraphs[0056] to[0059] of PATENT DOCUMENT 1.

The peptide according to the present invention encompasses a peptide having binding activity to VIPR2 even if the peptide has homology in which one to several amino acids are deleted, added and/or substituted in the amino acid sequence represented by the formula (1). As used herein, when "peptides in which one to several amino acids are deleted, added and/or substituted" is referred, the number of amino acids is not particularly limited as long as the peptide has a VIPR2 binding activity, but in one preferred embodiment, it is 1 to 5, and in one further preferred embodiment, it is 1 or 2. The deletion, addition, and/or substitution may occur at either the end or the middle of the peptide, and may occur at one or more locations.

Examples of the amino acid sequence in which one to several amino acids are deleted, added and/or substituted in the amino acid sequence include those having at least 50% or more, preferably 70% or more, more preferably 80% or more, particularly preferably 90% or more of the homology with the amino acid sequence when calculated using Basic Local Alignment Search Tool of National Center for Biological Information (BLAST) (e.g., by using default or initial setup parameters) or the like.

The peptide according to the present invention also encompasses various derivatives and/or modified forms thereof as long as they solve the problem of the present invention. Examples of such derivatives include: a peptide in which a saturated fatty chain is substituted with an unsaturated fatty chain; a peptide in which some of the atoms are substituted with other atoms including radioactive or non-radioactive isotope atoms; a peptide in which an amide bond is substituted with a thioamide bond (-NH-C(=S)-); a peptide in which an amide bond is substituted with alkene (-C=C-); a peptide in which an amide bond is substituted with alkyl (-C-C-); a peptide in which an amide bond is substituted with hydroxyethylene (-C(-OH)-C-); a peptide in which an amide bond is substituted with ester (-O-C(=O)-); a peptide in which an amide bond is substituted with alkene (-C=C-); a peptide in which an amide bond is substituted with (-C-NH-); and a peptide in which an amide bond is substituted with alkene (-C(=O)-C-). Examples of such modifications include, but are not limited to, a peptide in which a carbon at an α-position is disubstituted; a peptide in which an amide bond is N-alkylated; a peptide in which some of functional groups have been modified such as halogenated, cyanated, nitrated, hydroformylated, hydroxylated, aminated, deaminated, dehydroated, amidated, acetylated, methoxylated, prenylated, or alkylated (e.g., a peptide in which some of amino groups are acetylated, formylated, myristoylated, palmitoylated, pyroglutaminated, alkylated, or deaminated, a peptide in which some of carboxy groups are N-pyrrolidinylated or N-piperidinylated, or is an amide (such as amide, methylamide, ethylamide, p-nitroanilide, β-naphthylamide) or an ester (such as methyl ester, ethyl ester, thioester)); a peptide in which S is sulfoxide S(=O) or sulfone S(=O)₂; a peptide that is multimerized via a chemical linker; a peptide that is biotin-labeled; a peptide that is fluorescence-labeled; a peptide that is luminescence-labeled; and a peptide that is fused with an alkyl chain, polyethylene glycol, an antibody, a lectin, a sugar chain, an enzyme, a membrane penetrating peptide, a low molecular weight compound, a molecule that induces ubiquitination of a protein, or the like. Such modified peptides include, but are not limited to, those wherein the α - carbon of the peptide is disubstituted, the amide bond of the peptide is N-alkylated, some of the functional groups of the peptide are halogenated, cyanated, nitrated, oxonated, hydroxylated, aminated, deaminated, dehydrogenated, amidated, acetylated, methoxylated, prenylated, alkylated, etc., (e.g., some of the amino groups of the peptide are acetylated, formylated, myristoylated, palmitoylated, pyroglutamated, alkylated, deaminated, some of the carboxyl groups of the peptide are N-pyrrolidinylated, N-piperidinylated, amides (amide, methylamide, ethylamide, p-nitroanilide, β - naphthylamide, etc.), esters (methyl ester, ethyl ester, thioester, etc.), etc.), those wherein S of the peptide is sulfoxide S (= O) or sulfone S (= O) 2, those wherein the peptide is multimerized via a chemical linker, those wherein the peptide is biotin-labeled, those wherein the peptide is fluorescence-labeled, those wherein the peptide is luminescence-labeled, and those wherein the peptide is fused with an alkyl chain, polyethylene glycol, antibody, lectin, sugar chain, enzyme, membrane-permeable peptide, low molecular weight compound, or a molecule inducing ubiquitination of protein.

The peptide according to the present invention also encompasses salts of the peptide. As the salt of the peptide, a salt with a physiologically acceptable base or acid is used, and examples thereof include an addition salt of an inorganic acid (such as hydrochloric acid, hydrobromic acid, hydroiodic acid, sulfuric acid, phosphoric acid), an addition salt of an organic acid (such as p-toluenesulfonic acid, methanesulfonic acid, oxalic acid, p-bromophenylsulfonic acid, carboxylic acid, succinic acid, citric acid, benzoic acid, acetic acid), an inorganic base (such as ammonium hydroxide, an alkali or alkaline-earth metal hydroxide, a carbonate, a bicarbonate), and an addition salt of an amino acid.

The peptide according to the present invention may be a prodrug. A prodrug refers to a compound that is converted into the peptide according to the present invention by a reaction with an enzyme, gastric acid, or the like under physiological conditions in vivo, that is, a compound changed into the peptide according to the present invention by causing enzymatic oxidation, reduction, hydrolysis, or the like, or a compound changed into the peptide according to the present invention by causing hydrolysis or the like by gastric acid or the like.

Examples of the prodrug of the peptide according to the present invention include, but are not limited to, a compound of the peptide according to the present invention in which an amino group is acylated, alkylated, or phosphorylated (e.g., a compound of the peptide according to the present invention in which an amino group is eicosanoylated, alanylated, pentylaminocarbonylated, (5-methyl-2-oxo-1,3-dioxolen-4-yl)methoxycarbonylated, tetrahydrofuranylated, pyrrolidylmethylated, pivaloyloxymethylated, or tert-butylated); a compound of the peptide according to the present invention in which a hydroxy group is acylated, alkylated, phosphorylated, or borated (e.g., a compound of the peptide according to the present invention in which a hydroxy group is acetylated, palmitoylated, propanoylated, pivaloylated, succinylated, fumarylated, alanylated, or dimethylaminomethylcarbonylated); and a compound of the peptide according to the present invention in which a hydroxy group or carboxy group is esterified or amidated (e.g., a compound of the peptide according to the present invention in which a hydroxy group or a carboxy group is C₁₋₆ alkylesterified, phenylesterified, carboxymethylesterified, dimethylaminomethylesterified, pivaloyloxymethylesterified, ethoxycarbonyloxyethylesterified, phthalidylesterified, (5-methyl-2-oxo-1,3-dioxolen-4-yl)methylesterified, cyclohexyloxycarbonylethylesterified, or methylamidated). These compounds can be produced from the peptide according to the present invention by a method known per se.

The prodrug of the peptide according to the present invention may be one that is changed to the peptide according to the present invention under physiological conditions as described in "Development of Pharmaceuticals," volume 7, Molecular Design, page 163 to 198, published by Hirokawa Shoten 1990.

As used herein, the prodrug may form a salt, and examples of such a salt include those exemplified as the salt of the peptide according to the present invention.

The peptide according to the present invention may be a crystal, and either a single crystal form or a mixture of crystal forms is encompassed by the peptide according to the present invention. The crystal can be produced by crystallization by applying a crystallization method known per se.

The peptide according to the present invention may be a pharmaceutically acceptable co-crystal or a co-crystal salt. Here, co-crystals or co-crystal salts are crystalline substances comprising two or more unique solids at room temperature, each having different physical properties (e.g., structure, melting point, heat of fusion, hygroscopicity, solubility, and stability). Co-crystals, or co-crystal salts, can be produced according to co-crystallization methods known per se.

### (Composition containing Cyclic Peptide)

A cyclic peptide as one active ingredient of the present invention, a derivative or modified form thereof, can be used in the form of a composition as a medicine, a diagnostic agent, or a research reagent. The administration form of the composition (e.g., a pharmaceutical composition) is not particularly limited, and the composition may be administered orally or parenterally. Examples of the parenteral administration include transmucosal administration (nasal, transoral, transocular, transpulmonary, transvaginal, or transrectal administration), injection administration (intravenous, subcutaneous, intramuscular injections), and transdermal administration. The peptide in the composition can be modified in various ways in view of their propensity to be metabolized and excreted. For example, alkyl chains, polyethylene glycols, or sugar chains can be added to the peptide to increase its retention time in the bloodstream and reduce its antigenicity. In addition, bio-degradable polymeric compounds such as polylactic acid/glycol (PLGA), porous hydroxyapatite, liposomes, surface-modified liposomes, emulsions prepared with unsaturated fatty acids, nanoparticles, nanospheres, etc. can be used as sustained-release base agents, and the peptides can be encapsulated in them. When administered transdermally, a weak electric current can also be applied to the skin surface to penetrate the stratum corneum (iontophoresis method).

The above compositions may be used as the active ingredients as they are, or they may be formulated by adding pharmaceutically acceptable carriers, excipients, additives, etc. Examples of the dosage form include liquids (e.g., injections), dispersants, suspensions, tablets, rounds, powders, suppositories, sprays, fine granules, granules, capsules, syrups, lozenges, inhalants, ointments, eye drops, nasal drops, ear drops, and cataplasms. These formulations may be controlled release formulations (such as sustained-release microcapsules) such as fast release formulations or sustained-release formulations. The formulating can be carried out by an usual method using, for example, an excipient, a binder, a disintegrant, a lubricant, a dissolving agent, a solubilizing agent, a colorant, a flavoring agent, a stabilizing agent, an emulsifier, an absorption promoter, a surfactant, a pH adjusting agent, an antiseptic, and an antioxidant, as appropriate. Examples of ingredients used in formulating include, but are not limited to, purified water, saline solution, phosphate buffer solution, dextrose, glycerol, ethanol, and other pharmaceutically acceptable organic solvents, as well as animal and vegetable oils, lactose, mannitol, glucose, sorbitol, crystal cellulose, hydroxypropyl cellulose, starch, corn starch, silicic anhydride, aluminum magnesium silicate, collagen, polyvinyl alcohol, polyvinylpyrrolidone, carboxyvinyl polymer, sodium carboxymethyl cellulose, sodium polyacrylate, sodium alginate, water-soluble dextran, sodium carboxymethyl starch, pectin, methylcellulose, ethylcellulose, xanthan gum, gum arabic, tragacanth, casein, agar, polyethylene glycol, diglycerin, glycerin, propylene glycol, vaseline, paraffin, octyldodecyl myristate, isopropyl myristate, higher alcohols, stearyl alcohol, stearic acid, and human serum albumin. When the peptide is difficult to be transmucosally absorbed, the following may be used as absorption promoters for improving absorption of poorly absorbable drugs: surfactants such as polyoxyethylene lauryl ethers, sodium lauryl sulfate, and saponin; bile salts such as glycocholic acid, deoxycholic acid, and taurocholic acid; chelating agents such as EDTA and salicylic acids; fatty acids such as caproic acid, capric acid, lauric acid, oleic acid, linoleic acid, and mixed micelles; an enamine derivative, an N-acylcollagen peptide, N-acylamino acid, cyclodextrins, chitosans, and a nitric oxide donor.

A pill or tablet can also be coated with a sugar coating, a gastrosoluble, or an enteric substance. The injection may contain distilled water for injection, physiological saline, propylene glycol, polyethylene glycol, vegetable oils, alcohols, and the like. Humectants, emulsifiers, dispersants, stabilizing agents, dissolving agents, solubilizing agents, antiseptics, and the like may also be added. If necessary, additives such as normal antiseptics, antioxidants, colorants, sweetening agents, adsorbents, and humectants can be used in appropriate amounts.

Since the composition of the present invention has action of suppressing cancer metastasis, it can be administered to a patient with tumor metastasis and a patient with high risk of tumor metastasis. Thus, the composition may be administered to a subject prior to or after the occurrence of tumor metastasis. The application target in the subject is not particularly limited as long as it is cells, but is preferably tumor cells. The type of cancer is not limited, and examples thereof include head and neck cancer, gastric cancer, colon cancer, rectal cancer, large bowel cancer, liver cancer, gallbladder/bile duct cancer, pancreatic cancer, lung cancer, breast cancer, bladder cancer, prostate cancer, uterine cancer, oral cancer, pharyngeal cancer, throat cancer, tongue cancer, esophageal cancer, renal cancer, and ovarian cancer.

The composition of the present invention may be used in combination with other medicines and therapies useful for the above diseases, such as various types of chemotherapies, surgical treatments, and radiotherapy.

When the composition of the present invention is administered to a mammal (e.g., human, mouse, rat, guinea pig, rabbit, dog, horse, monkey, pig), particularly human, the dosage varies depending on the symptom, the age, sex, body weight, and sensitivity difference of the subject, as well as the administration method, administration interval, type of active ingredient, and type of formulation, and is not particularly limited, but for example, 30 µg to 1000 mg, 100 µg to 500 mg, or 100 µg to 100 mg can be administered once or in several divided doses.

The following examples are merely illustrative, and are intended only to describe the present invention in detail together with the above-described embodiments, and are not intended to limit the present invention. A person skilled in the art can change the present invention to various ways without departing from the meaning of the present invention, and such changes are also included in the scope of the present invention.

### Examples

### (Peptide Synthesis)

The chemical synthesis of all peptides used in the present example was outsourced to SCRUM Inc. (Tokyo, Japan), and a standard solid phase synthesis method using a 9-fluorenylmethoxycarbonyl group (Fmoc group) as a protecting group of an α-amino group was performed by an automatic synthesizer SyroII (manufactured by Biotage). The C-terminal side-chain protected amino acid-resin was placed in the synthesis column, and the column was set in the synthesizer. Subsequently, 1-[bis(dimethylamino)methylene]-1H-1,2,3-triazolo[4,5-b]pyridinium 3-oxidehexafluorophosphate (HATU)/diisopropylethylamine (DIEA) was added to the next amino acid protected with the Fmoc group to activate the next amino acid, and the mixture was placed in a column and allowed to react. After the reaction was completed, the resultant was washed and the Fmoc group was deprotected using 20% piperidine. By repeating this step, the peptide chain was extended, the Fmoc group of the final amino acid was deprotected, and then the peptide resin was removed from the synthesizer.

Cyclization of the peptides was performed by referring to procedures described in, for example, the following: Biopolymers. 2016 106(6), 843-852; Chem Soc Rev. 2015, 44(1) 91-102; Nat Chem. 2014, 6(11) 1009-1016. As an example, cyclization of the Seq-1 and the Seq-10 is shown below. The linear side-chain-protected peptide resin was swelled with dimethylformamide (DMF) and reacted in a 2% hydrazine solution for 5 minutes to 10 minutes, thereby deprotecting the protecting group (Dde) of the side chain of lysine and the protecting group (ODmab) of the side chain of aspartic acid. Then, coupling reagents Oxima Pure and diisopropylcarbodiimide (DIC) were added and reacted at 50°C for 3 hours. After washing the resin, TFA was added to deprotect the protecting group of the thiol group, and at the same time, a monocyclic peptide was cut out from the resin. The monocyclic peptide was purified by RT-HPLC using a SunFire C18 column (10×150 mm) (manufactured by Waters Corporation) and then freeze-dried. The monocyclic peptide was dissolved in a mixture of Tris-HCl buffer (pH 8.5) and acetonitrile, DMSO was then added to the resultant solution, and the resultant was stirred at room temperature for 36 hours to cyclize the peptide through a disulfide bond. Seq-1 and Seq-10, the bicyclic peptides, were purified by RT-HPLC using a SunFire C18 column (10x150 mm) (manufactured by Waters Corporation) and then freeze-dried. The molecular weight of the finally obtained peptide was measured using microflex (Bruker), and the target product was identified.

The theoretical molecular weight, the measured molecular weight, the purity, the cyclization type, and the sequence of the peptide synthesized in this example are shown in Table 1. Among them, the amino acid sequences of Seq-1 and Seq-10 are shown below. In Table 1 and the following amino acid sequences, amino acids without the D-form notation indicate the L-form.

**[Table 1]**

| Name | Calcd MW | Obsvd MW | Voltage polarity | Purity (%) | Cyclization |
|---|---|---|---|---|---|
| Seq-1 (SEQ ID NO: 1) | 1508.8 | 1508.8 | NEG | 95.9 | S-S(-10)/amide(7-11) |
| Seq-10 (SEQ ID NO: 2) | 1558.8 | 1558.7 | NEG | 100.0 | S-S(1-10)/amide(7-11) |

| | | | | | |
|---|---|---|---|---|---|
| Seq-1: c(Mpa-Pro-Pro-Tyr-Leu-Peo-c[Lys-Tyr-Leu-Cys)-Asp]-Leu-Ile-NH₂ (SEQ ID NO: 1) Seq-10: Ac-c[Cys¹-Pro²-Pro³-Tyr⁵-Leu⁶-c(Lys⁷-Tyr⁸-Leu⁹-Cys¹⁰]-Asp¹¹)-Leu¹²-Ile¹³-NH₂ (SEQ ID NO: 2) | | | | | |

Seq-1 and Seq-10 are peptides obtained by removing three residues (Leu-Arg-Ser) at the C-terminal of VIpep-3 (see NON-PATENT DOCUMENT 1) and performing bicyclization by an S-S bond between positions 1 and 10 and an amide bond between positions 7 and 11.

### (Reference Example) Analysis of Migration Mechanism of Cancer Cell by VIPR2 Signal

### Materials and Methods

### <Plasmid and siRNA>

pCMV6-AN-Myc-DDK vector (PS100016) and negative control siRNA (S10C-0600) were purchased from Cosmo Bio Inc. The pEGFP-N2 vector was purchased from Takara Clontech. The pCMV6-VIPR2-Myc-DDK plasmid was constructed from the pCMV6-AN-Myc-DDK vector and the VIPR2 cDNA. The VIPR2-Myc region was cloned from the plasmid obtained into a pEGFP-N2 vector. Human VIPR2-siRNA (si1, 3024813653-000080 and - 000090; si2, 3024813653-000020 and -000030; si3, 3024813653-000050 and -000060) was purchased from Sigma-Aldrich.

### <Antibody>

Anti-GAPDH antibody (#2118), anti-WAVE2 antibody (#3659), anti-pan AKT antibody (#4691), anti-phosphorylated AKT antibody (Ser473; #4060), and anti-phosphorylated AKT antibody (Thr308; #2965) were purchased from Cell Signaling Technology, Inc.

### <Production of VIPR2-EGFP Overexpressing Cells>

MCF-7 and MDA-MB-231 cell lines were purchased from the JCRB Cell Bank (National Institutes of Biomedical Innovation, Health and Nutrition). Lipofectamine 3000 (Invitrogen) was used to transfect control siRNA and VIPR2 siRNA according to the manufacturer's recommendations. MCF-7 and MDA-MB-231 cells were transfected with an expression vector encoding VIPR2-EGFP or a control EGFP expression vector, and cultured in the presence of 1 mg/mL G418 (Nacalai Tesque) for 14 days to establish a cell line stably expressing VIPR2-EGFP or EGFP.

### <Western Blotting>

Western blotting was performed according to a previously described method (Asano et al. 2014, Biol. Open3, 463-474; Asano et al. 2019, Sci. Rep. 9, 12729).

### <Random Migration Test and Transwell Migration Test>

MCF-7 cells (1.5×10⁴ cells) were seeded in a 35 mm culture dish with serum-free medium, cultured until adhesion, and then stimulated with 100 nM VIP (Cayman Chemical Company). For the migration assay, cells were monitored by live cell imaging (BZ-X800; Keyence Corporation) every hour at 37°C for 12 hours using a thermoplate (manufactured by Tokai Hit Co., Ltd.). Tracking analysis of cells was performed using Image-Pro Premier ver. 9.4 (Media Cybernetics, Inc.).

In addition, the measurement was performed according to the method described above (Asano et al. 2012, Mol. Cell Biol. 32, 3242-3252) using a Boyden chamber. To each well, 600 µL of a serum-free culture solution containing 200 nM VIP was added. Cells (1×10⁴ cells) were suspended in 100 µL of a serum-free culture solution and added to the insert. The cells were allowed to migrate for 30 or 48 hours at 37°C. Non-mobile cells adhering to the upper surface of the membrane were removed, and mobile cells adhering to the lower surface were fixed with 4% paraformaldehyde and counted with a fluorescence microscope.

### <Mouse Intraperitoneal Metastasis Test>

Intraperitoneal metastasis of MDA-MB-231 breast cancer cells was evaluated by modifying the method performed by Cabral et al., using HeLa cells (Cabral et al., 2007, J. Control Release. 121, 146-155). Briefly, 5-week-old age-matched female nude mice (BALB/c-nu; Charles River Laboratories Japan, Inc.) were intraperitoneally inoculated with MDA-MB-231 cells stably expressing VIPR2-EGFP or MDA-MB-231 cells stably expressing EGFP (6×10⁶ cells/200 µL) (n=3/group). Tumor growth and metastasis were visualized with live animal imaging using NightOWL (from Berthold Technologies) and monitored weekly for 6 weeks.

### <Statistical Analysis>

GraphPad Prism was used for statistical analysis. Dunn's multiple comparison test was used in addition to the nonparametric Mann-Whitney U test (for two groups) and Kruskal-Wallis test (for two or more groups). When the p value was less than 0.05, it was determined to be statistically significant.

### (Results)

### <VIP-VIPR2 Signal Promotes PI3K/PI(3,4,5)P₃ Pathway in MCF-7 and MDA-MB-231 Breast Cancer Cells>

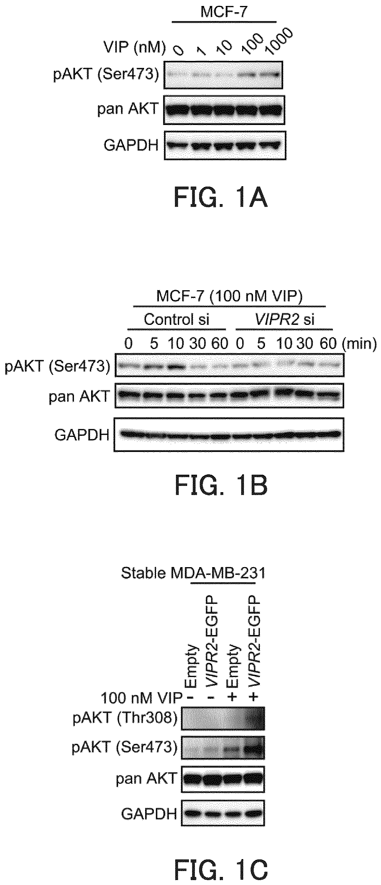

To investigate whether VIP is involved in cell movement via the PI3K/PI(3,4,5)P₃ pathway, phosphorylation of AKT, a downstream signal of the PI3K/PI(3,4,5)P₃ pathway in cancer cells, was assessed by Western blotting. As shown in FIG. 1A, when MCF-7 cells were starved for 3 hours and then stimulated with 0 nM to 1000 nM of VIP for 10 minutes, AKT was phosphorylated in a VIP addition concentration-dependent manner. As shown in FIG. 1B, this phosphorylation of AKT was strongly suppressed by siRNA against VIPR2. As shown in FIG. 1C, this phosphorylation of AKT was significantly increased in MDA-MB-231 cells stably expressing VIPR2-EGFP. All experiments were independently performed at least three times, and similar data were obtained. Representative images are shown in FIGS. 1A to 1C. These results indicate that activation of PI3K/PI(3,4,5)P₃ pathway induced by VIP is mediated by VIPR2 in breast cancer cells such as MCF-7 and MDA-MB-231.

### <VIP-VIPR2 Signal Promotes Cell Migration of MCF-7 Breast Cancer Cells>

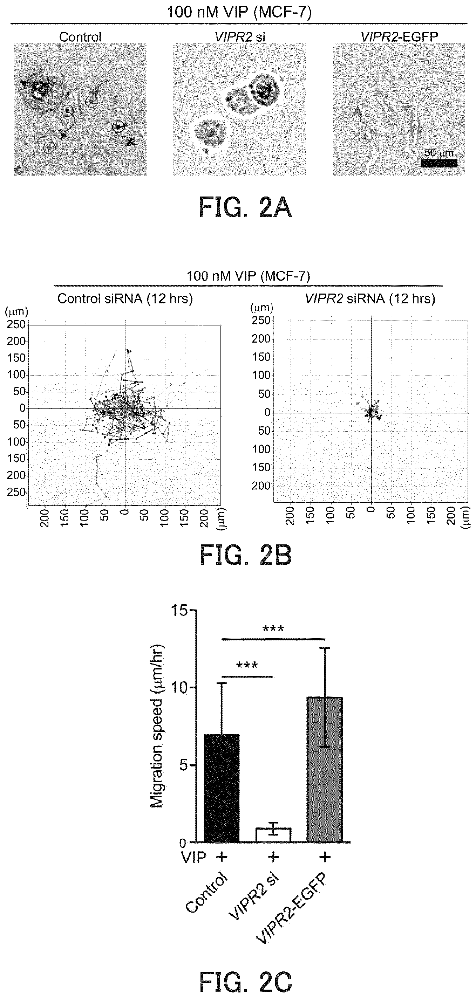

To investigate whether VIPR2 expression affects cancer cell motility, cell migration of MCF-7 cells was examined. As shown in FIG. 2A, in the presence of 100 nM VIP, the movement of MCF-7 cells, occurring with the lapse of culture time, was strongly suppressed by siRNA against VIPR2. On the other hand, in the MCF-7 cells stably expressing VIPR2-EGFP, in addition to the movement of the cells, the morphology of the cells was changed, and pseudopodium was remarkably observed. FIG. 2B shows a result of analyzing and graphing a track plot of MCF-7 cells transfected with control siRNA and VIPR2 siRNA. With start points of individual cells being set to the middle of the figure, it is shown that movement of MCF-7 cells was strongly suppressed by siRNA against VIPR2. FIG. 2C is a bar graph showing a comparison of the moving speeds. The moving speed of MCF-7 cells was strongly suppressed by siRNA against VIPR2, whereas the moving speed of MCF-7 cells stably expressing VIPR2-EGFP was significantly increased. Data are shown as mean ± SD. ***: p<0.001 between the displayed groups (Kruskal-Wallis test followed by Dunn's multiple comparison test).

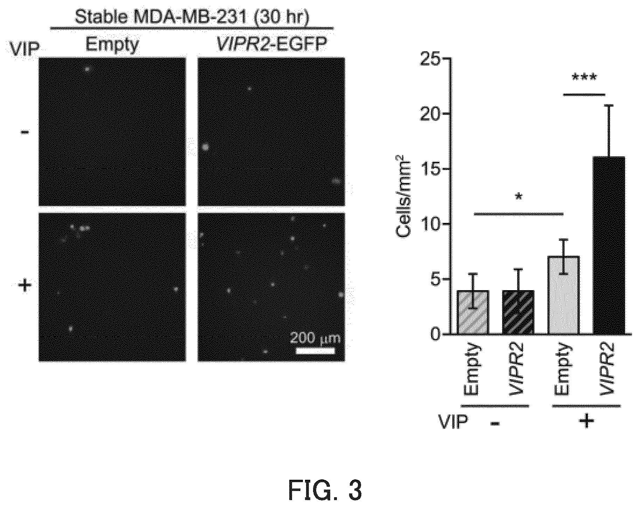

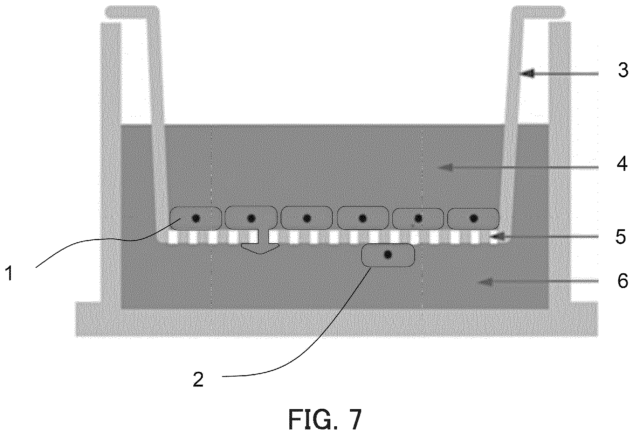

The movement ability of MDA-MB-231 cells was evaluated using the transwell chamber assay shown in FIG. 7. Cells were suspended in serum-free media (1×10⁴ cells/100 mL). A serum-free medium 6 containing 200 nM VIP was added to each well in an amount of 600 µL, and an insert 3 having 3 µm pores 5 was immersed in the well. Then, the suspended cells 1 were added to the insert 3, and cultured under conditions of 37°C and 5% CO₂. After 30 hours, cells were fixed, cells on the surface of the insert 3 were removed, and then fluorescence of EGFP of cells 2 that had moved to the rear surface in response to VIP was detected with a fluorescence microscope. The number of the cells that had moved to the rear surface of the insert was graphed based on the fluorescence image. FIG. 3 shows the results of measurement of movements of MDA-MB-231 cells stably expressing EGFP or VIPR2-EGFP using a transwell chamber assay. VIP (200 nM) was added to the well, the cells were added to the insert and incubated at 37°C for 30 hours, and cells that had moved to the rear side of the insert were quantified and shown graphically. In the presence of VIP, the number of cells that had moved to the back side of the insert increased, and the movement of the cells expressing VIPR2-EGFP was further remarkably enhanced. Data are shown as mean ± SD. *: p<0.05, ***: p<0.001 between displayed groups (Kruskal-Wallis test followed by Dunn's multiple comparison test). These results indicate that cell movement induced by VIP is promoted in a VIPR2 expression level-dependent manner.

### <Overexpression of VIPR2 Results in Formation of WAVE2-Rich Lamellipodia>

MDA-MB-231 cells (2×10⁵ cells) stably expressing EGFP or VIPR2-EGFP were seeded on collagen-coated cover glass and incubated under conditions of 37°C and 5% CO₂. After 24 hours, the cells were washed with serum-free medium, and fresh serum-free medium was added to incubate the cells (under conditions of 37°C and 5% CO₂). After 3 hours, the medium was replaced with serum-free medium containing 100 nM VIP, and incubation for 30 minutes was performed. The cells were fixed with 4% paraformaldehyde, and immunostained using an anti-WAVE2 antibody. The surface area of a WAVE2-rich region immediately below the cell membrane was measured from a fluorescently-stained image of the same and graphed.

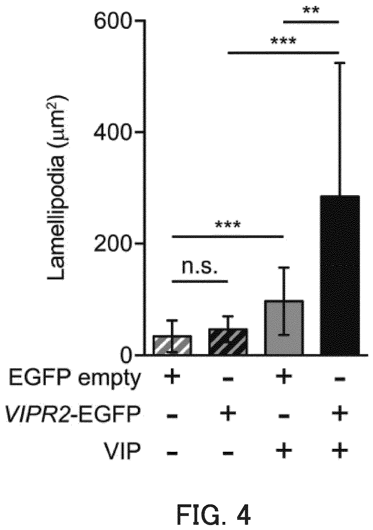

The results are shown in FIG. 4. The bar graph shows the areas of lamellipodia. Data are shown as mean ±SD. **: p<0.01, ***: p<0.001, between indicated groups (Kruskal-Wallis test and Dunn's multiple comparison test). "n.s." indicates not statistically significant. The VIP stimulation expanded the WAVE2-rich area of lamellipodia of MDA-MB-231. The MDA-MB-231 cells stably expressing VIPR2-EGFP had more pronounced area expansion of lamellipodia compared to the control MDA-MB-231 cells expressing EGFP.

### <Overexpression of VIPR2 Promotes Tumor Metastasis in vivo>

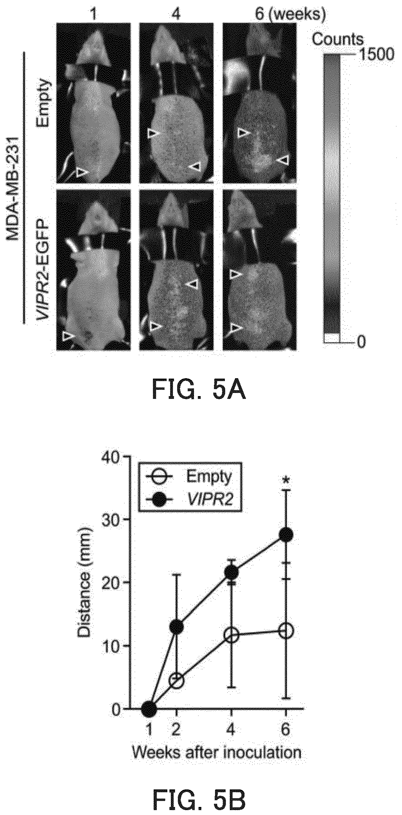

In order to examine whether VIPR2 plays a role in tumor metastasis and diffusion in vivo, nude mice were intraperitoneally inoculated with MDA-MB-231 cells stably expressing EGFP or VIPR2-EGFP. In vivo fluorescence imaging was performed using NightOWL every other week to observe systemic migration of cancer cells. The results are shown in FIG. 5A. Panels are fluorescence images of MDA-MB-231 cells expressing EGFP or VIPR2-EGFP in vivo at weeks 1, 4, and 6. The graph in FIG. 5B shows the distances (distance from the site of administration to the farthest cell population) regarding the portion transplanted at week 1 and the next confluent portion (arrowhead) in the respective indicated weeks.

These results indicate that the MDA-MB-231 cells stably expressing VIPR2-EGFP moved distantly in the abdominal cavity compared to the MDA-MB-231 cells stably expressing EGFP. From the above results, it was revealed that overexpression of VIPR2 promotes movement of tumor cells in vivo.

### <VIPR2-Selective Antagonist KS-133 inhibits Activation of PI3K/PI(3,4,5)P₃

Pathway by VIP in MDA-MB-231 Cells Stably Expressing VIPR2-EGFP> Phosphorylation of AKT by VIP in MDA-MB-231 cells stably expressing VIPR2-EGFP was evaluated in the presence and absence of KS-133. KS-133 strongly suppressed phosphorylation of AKT induced by VIP in an addition concentration-dependent manner. KS-133 did not affect the expression of AKT.

### <VIPR2-Selective Antagonist KS-133 Suppresses Migration of MDA-MB-231 Breast Cancer Cells Stably Expressing VIPR2-EGFP>

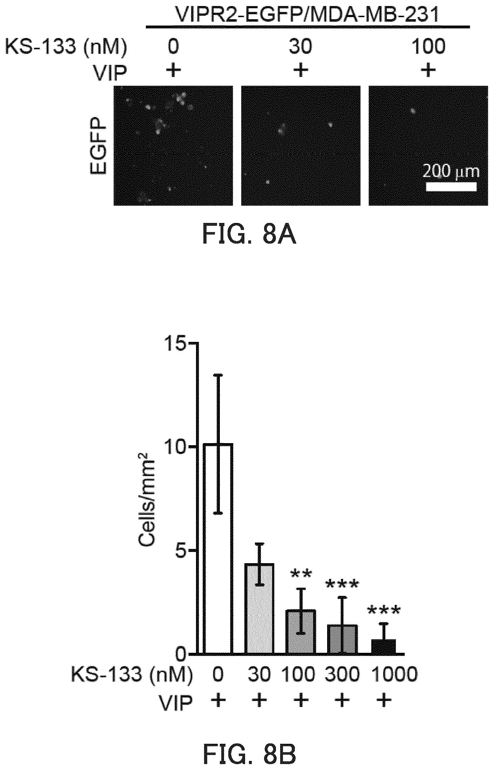

Using the transwell shown in FIG. 7, the effect of KS-133 on migration of MDA-MB-231 breast cancer cells stably expressing VIPR2-EGFP was examined. MDA-MB-231 cells stably expressing VIPR2-EGFP were suspended in serum-free medium containing KS-133 at each of the concentrations of 0 nM to 1000 nM (1×10⁴ cells/100 µL). A serum-free medium 6 containing 200 nM VIP and KS-133 at each of the concentrations was added to each well in an amount of 600 µL, and an insert 3 having 3 µm pores 5 was immersed in the well. Then, the suspended cells 1 were added to the insert 3, and incubated under conditions of 37°C and 5% CO₂. After 48 hours, the cells were fixed, cells on the surface of the insert 3 were removed, and then fluorescence of EGFP of cells 2 that had moved to the rear surface in response to induction by VIP was detected with a fluorescence microscope. The number of the cells that had moved to the rear surface of the insert was graphed based on the fluorescence image. The results are shown in FIG. 8.

FIG. 8A is a fluorescence micrograph showing migrated cells. FIG. 8B and Table 2 show the number of migrated cells when each concentration of KS-133 was added. VIP-induced cell migration of MDA-MB-231 cells stably expressing VIPR2-EGFP was suppressed by the addition of KS-133 (as a result of Kruskal-Wallis test and Dunn's multiple comparison test, the presence of KS-133, only when 100 nM or more of KS-133 was added, showed a significant difference compared to the absence of KS-133).

**[Table 2]**

| | | | | | |
|---|---|---|---|---|---|
| KS-133 addition concentration (nM) | 0 | 30 | 100 | 300 | 1000 |
| Mean cell count (Cells/mm²) | 10.13 | 4.35 | 2.08 | 1.39 | 0.78 |
| Standard deviation | 3.34 | 0.99 | 1.07 | 1.34 | 0.70 |

### <Cell Proliferation Suppression Effect of Addition of KS-133, Selective VIPR2 Antagonist, in MCF-7 Cells Overexpressing VIPR2>

This effect was confirmed. The cell proliferation test was performed according to NON-PATENT DOCUMENT 3. MCF-7 cells overexpressing VIPR2-EGFP were seeded in a 35 mm culture dish at a density of 1×10⁵ cells/dish for 6 hours, and the culture medium was replaced with a fresh medium containing 2% FBS, 100 nM VIP, and 0 nM or 100 nM KS-133 (day 0). Cells were observed under a BZ-X800 fluorescence microscope (Keyence, Kyoto, Japan) and the number of cells was counted every 24 hours for up to 4 days by trypan blue dye staining using a hemacytometer (timecourse experiment).

To MCF-7 cells overexpressing VIPR2-EGFP, KS-133 as a VIPR2 antagonist was added (NON-PATENT DOCUMENT 2), and the effect of proliferation of the cells was confirmed. The confirmed results are shown in FIG. 9 and Table 3. In the MCF-7 cells overexpressing VIPR2, cell proliferation was significantly suppressed from Day 2 of culture or later by the addition of KS-133. In the control group, cell proliferation was confirmed over time. This confirmed result revealed that the VIP-VIPR2 signal causes proliferation of breast cancer cells.

**[Table 3]**

| | Control group | | KS-133 group | |
|---|---|---|---|---|
| Day | Number of cells (mean value) | Standard deviation | Number of cells (mean value) | Standard deviation |
| 0 | 1.0 × 10⁵ | 0 | 1.0 × 10⁵ | 0 |
| 1 | 1.5 × 10⁵ | 0.9309 | 0.85 × 10⁵ | 0.2517 |
| 2 | 1.45 × 10⁵ | 0.3416 | 0.65 × 10⁵ | 0.2517 |
| 3 | 1.95 × 10⁵ | 0.9849 | 0.45 × 10⁵ | 0.3786 |
| 4 | 3.2 × 10⁵ | 0.6325 | 0.2 × 10⁵ | 0.1633 |

### INDUSTRIAL APPLICABILITY

The composition containing the cyclic peptide according to the present invention may be used as a pharmaceutical such as a cancer metastasis suppressor.

### DESCRIPTION OF REFERENCE CHARACTERS

- 1: Cell
- 2: Cell Having Induced by VIP and Moved
- 3: Insert
- 4: KS-133 (Present or Absent)/Serum-Free Medium
- 5: Pore (3 µm)
- 6: 200 nM VIP + KS-133 (Present or Absent)/Serum-Free Medium

## Claims

1. A composition for suppressing cancer metastasis, the composition comprising:
a cyclic peptide having an amino acid sequence represented by the formula (1):
c[X¹-Pro²-X³-Tyr⁴-Leu⁵-Pro⁶-c(X⁷-X⁸-Leu⁹-Cys¹⁰]-X¹¹)-X¹²-X¹³ (1)
where X¹ represents cysteine, Mpa (3-mercaptopropionic acid), or D-cysteine,
X³ represents N-methylated glycine, N-methylated alanine, 2-azetidine-2-carboxylic acid, proline, hydroxyproline, 3,4-dehydroproline, pipecolic acid, serine, or lysine,
X⁸ represents tyrosine, proline, or arginine,
X⁷ and X¹¹ represent any combination of lysine and aspartic acid, ornithine and glutamic acid, aspartic acid and lysine, glutamic acid and ornithine, lysine and glutamic acid, or glutamic acid and lysine,
X¹² and X¹³ each independently represent leucine, isoleucine, or norleucine, and
X¹ and Cys¹⁰ form a disulfide bond between their side chains, and X⁷ and X¹¹ form an amide bond between their side chains, whereby the peptide of the formula (1) has two cyclic structures in a molecule, and an N-terminus amino group and a C-terminus carboxy group can be modified or deleted;
a derivative or a modified form of the cyclic peptide, or a pharmacologically acceptable salt thereof.

2. The composition of claim 1, wherein, in the cyclic peptide,
X¹ represents cysteine,
X³ represents proline or serine,
X⁷ represents lysine,
X⁸ represents tyrosine,
X¹¹ represents aspartic acid, and
X¹² and X¹³ each independently represent leucine, isoleucine, or norleucine, the N-terminus amino group is acetylated, and the C-terminal carboxy group is amidated.

3. The composition of claim 1 or 2, wherein the composition is for use in treatment of a subject including a cancer cell in which PI3K/AKT signaling pathway is activated.

4. The composition of claim 3, wherein the composition suppresses phosphorylation of AKT in the cancer cell.
